# EUROPEAN PATENT SPECIFICATION

(11) **EP 2 569 329 B1**
(45) Date of publication and mention of the grant of the patent: **12.10.2016**
(21) Application number: 11721462.7
(22) Date of filing: 11.05.2011
(51) Int. Cl.: C07K 14/415, C12N 15/82

(54) **GROWTH PROMOTING FUSION PROTEINS**
WACHSTUMSFÖRDERNDE FUSIONSPROTEINE
PROTÉINES DE FUSION FAVORISANT LA CROISSANCE

(30) Priority: 11.05.2010 US 395398 P; 11.05.2010 GB 201007834
(43) Date of publication of application: 20.03.2013
(73) Proprietor: VIB vzw, 9052 Gent, Zwijnaarde (BE); Universiteit Gent, 9000 Gent (BE); Regents of the University of Minnesota, St. Paul, Minnesota 55114 (US)
(72) Inventor: INZÉ, Dirk, Gustaaf, B-9310 Moorsel - Aalst (BE); GONZALEZ, Nathalie, B-9820 Merelbeke (BE); GRAY, William M., St. Paul Minnesota 55113 (US); SPARTZ, Angela K., Falcon Heights Minnesota 55113 (US)
(74) Representative: Demolder, Jan Albert
(86) International application number: PCT/EP2011/057589
(87) International publication number: WO 2011/141499

(56) References cited:
- WO-A1-2011/036160
- PARK ET AL: "Functional characterization of a small auxin-up RNA gene in apical hook development in Arabidopsis", PLANT SCIENCE, ELSEVIER IRELAND LTD, IE, vol. 172, no. 1, 11 November 2006 (2006-11-11), pages 150-157, XP005760191, ISSN: 0168-9452, DOI: 10.1016/J.PLANTSCI.2006.08.005
- KANT SURYA ET AL: "SAUR39, a small auxin-up RNA gene, acts as a negative regulator of auxin synthesis and transport in rice.", PLANT PHYSIOLOGY OCT 2009 LNKD- PUBMED:19700562, vol. 151, no. 2, October 2009 (2009-10), pages 691-701, XP000002658465, ISSN: 0032-0889
- KNAUSS S ET AL: "The auxin-induced maize gene ZmSAUR2 encodes a short-lived nuclear protein expressed in elongating tissues", JOURNAL OF BIOLOGICAL CHEMISTRY 20030727 AMERICAN SOCIETY FOR BIOCHEMISTRY AND MOLECULAR BIOLOGY INC. US, vol. 27, no. 26, 27 July 2003 (2003-07-27) , pages 23936-23943, XP000002658435, DOI: DOI:10.1074/JBC.M212585200
- JAIN ET AL: "Genome-wide analysis, evolutionary expansion, and expression of early auxin-responsive SAUR gene family in rice (Oryza sativa)", GENOMICS, ACADEMIC PRESS, SAN DIEGO, US, vol. 88, no. 3, 23 August 2006 (2006-08-23), pages 360-371, XP005742406, ISSN: 0888-7543, DOI: 10.1016/J.YGENO.2006.04.008
- HAGEN G ET AL: "Auxin-responsive gene expression: Genes, promoters and regulatory factors", PLANT MOLECULAR BIOLOGY 2002 KLUWER ACADEMIC PUBLISHERS NL, vol. 49, no. 3-4, 2002, pages 373-385, XP000002658436, DOI: DOI:10.1023/A:1015207114117
- Spartz A. K., Lee S. H., Gray W. M.: "Overexpression of stabilized SAUR fusion proteins promote cell expansion", , 6 August 2011 (2011-08-06), XP000002658437, Retrieved from the Internet: URL:http://abstracts.aspb.org/pb2011/publi c/P16/P16049.html [retrieved on 2011-09-07]
- ANGELA K. SPARTZ ET AL: 'The SAUR19 subfamily of SMALL AUXIN UP RNA genes promote cell expansion' THE PLANT JOURNAL vol. 70, no. 6, 01 June 2012, pages 978 - 990, XP055202640 DOI: 10.1111/j.1365-313X.2012.04946.x ISSN: 0960-7412

### SEQUENCE LISTING

<110> VIB VZW UNIVERSITEIT GENT REGENTS OF THE UNIVERSITY OF MINNESOTA
<120> GROWTH PROMOTING FUSION PROTEINS
<130> DI/SAUR/341
<150> GB 1007834.3
   <151> 2010-05-11
<150> US 61/395,398
   <151> 2010-05-11
<160> 6
<170> PatentIn version 3.5
<210> 1
   <211> 90
   <212> PRT
   <213> Arabidopsis thaliana
<400> 1
   Met Ala Phe Val Arg Ser Leu Leu Gly Ala Lys Lys Ile Leu Ser Arg
   1               5                   10                  15
   Ser Thr Ala Ala Gly Ser Ala Ala Pro Lys Gly Phe Leu Ala Val Tyr
               20                  25                  30
   Val Gly Glu Ser Gln Lys Lys Arg Tyr Leu Val Pro Leu Ser Tyr Leu
           35                  40                  45
   Ser Gln Pro Ser Phe Gln Ala Leu Leu Ser Lys Ser Glu Glu Glu Phe
       50                  55                  60
   Gly Phe Ala His Pro Met Gly Gly Leu Thr Ile Pro Cys Pro Glu Asp
   65                  70                  75                  80
   Thr Phe Ile Asn Val Thr Ser Arg Leu Gln
                   85                  90
<210> 2
   <211> 88
   <212> PRT
   <213> Arabidopsis thaliana
<400> 2
   Met Ala Leu Val Arg Ser Leu Leu Gly Ala Lys Lys Ile Leu Ser Arg
   1               5                   10                  15
   Ser Thr Ala Ser Ala Ala Pro Lys Gly Phe Leu Ala Val Tyr Val Gly
               20                  25                  30
   Glu Ser Gln Lys Lys Arg Tyr Leu Val Pro Leu Ser Tyr Leu Ser Gln
           35                  40                  45
   Pro Ser Phe Gln Ala Leu Leu Ser Lys Ser Glu Glu Glu Phe Gly Phe
       50                  55                  60
   Asp His Pro Met Gly Gly Leu Thr Ile Pro Cys Pro Glu Asp Thr Phe
   65                  70                  75                  80
   Ile Asn Val Thr Ser Arg Leu Gln
                   85
<210> 3
   <211> 90
   <212> PRT
   <213> Arabidopsis thaliana
<400> 3
   Met Ala Leu Val Arg Ser Leu Leu Val Ala Lys Lys Ile Leu Ser Arg
   1 5 10 15
   Ser Ala Ala Ala Val Ser Ala Pro Pro Lys Gly Phe Leu Ala Val Tyr
   20 25 30
   Val Gly Glu Ser Gln Lys Lys Arg Tyr Leu Val Pro Leu Ser Tyr Leu
   35 40 45
   Asn Gln Pro Ser Phe Gln Ala Leu Leu Ser Lys Ser Glu Glu Glu Phe
   50 55 60
   Gly Phe Asp His Pro Met Gly Gly Leu Thr Ile Pro Cys Pro Glu Asp
   65 70 75 80
   Thr Phe Ile Asn Val Thr Ser Arg Leu His
   85 90
<210> 4
   <211> 90
   <212> PRT
   <213> Arabidopsis thaliana
<400> 4
   Met Ala Phe Val Arg Ser Leu Leu Gly Ala Lys Lys Ile Leu Ser Arg
   1 5 10 15
   Ser Thr Gly Ala Gly Ser Ala Ala Pro Lys Gly Phe Leu Ala Val Tyr
   20 25 30
   Val Gly Glu Ser Gln Lys Lys Arg Tyr Leu Val Pro Val Ser Tyr Leu
   35 40 45
   Asn Gln Pro Ser Phe Gln Ala Leu Leu Ser Lys Ser Glu Glu Glu Phe
   50 55 60
   Gly Phe Asp His Pro Met Gly Gly Leu Thr Ile Pro Cys Pro Glu Asp
   65 70 75 80
   Thr Phe Ile Asn Val Thr Ser Arg Leu Gln
   85 90
<210> 5
   <211> 8
   <212> PRT
   <213> Arabidopsis thaliana
<220>
   <221> MISC_FEATURE
   <222> (1)..(1)
   <223> Xaa can be E, K or R
<220>
   <221> MISC_FEATURE
   <222> (3)..(3)
   <223> Xaa can be H, N, F, Y or S
<220>
   <221> MISC_FEATURE
   <222> (4)..(4)
   <223> Xaa can be V, L or F
<220>
   <221> MISC_FEATURE
   <222> (5)..(5)
   <223> Xaa can be P or A
<220>
   <221> MISC_FEATURE
   <222> (7)..(7)
   <223> Xaa can be Y, S or C
<400> 5
   Xaa Gly Xaa Xaa Xaa Val Xaa Val
   1               5
<210> 6
   <211> 11
   <212> PRT
   <213> Arabidopsis thaliana
<220>
   <221> MISC_FEATURE
   <222> (3)..(3)
   <223> Xaa can be R, E, K or S
<220>
   <221> MISC_FEATURE
   <222> (4)..(4)
   <223> Xaa can be R, K, E or D
<220>
   <221> MISC_FEATURE
   <222> (5)..(5)
   <223> Xaa can be A or V
<220>
   <221> MISC_FEATURE
   <222> (6)..(6)
   <223> Xaa can be A or E
<220>
   <221> MISC_FEATURE
   <222> (7)..(7)
   <223> Xaa can be Q or E
<220>
   <221> MISC_FEATURE
   <222> (9)..(9)
   <223> Xaa can be Y or F
<220>
   <221> MISC_FEATURE
   <222> (11)..(11)
   <223> Xaa can be Y or F
<400> 6
   Leu Leu Xaa Xaa Xaa Xaa Xaa Glu Xaa Gly Xaa
   1               5                   10

## Description

The present invention relates to fusion proteins of polypeptides of the SAUR family that promote plant growth. More specifically, it relates to fusion proteins of polypeptides of the SAUR family fused to a heterologous polypeptide, fused at the N-terminal end of the SAUR polypeptide. Even more specifically, it relates to the use of said fusion protein in combination with other recombinant genes to promote plant growth. Said combination of genes can be expressed in a transgenic plant to obtain an additive or even synergistic effect.

The demand for more plant derived products has spectacularly increased. In the near future the challenge for agriculture will be to fulfill the growing demands for feed and food in a sustainable manner. Moreover plants start to play an important role as energy sources. To cope with these major challenges, a profound increase in plant yield will have to be achieved. Biomass production is a multi-factorial system in which a plethora of processes are fed into the activity of meristems that give rise to new cells, tissues, and organs. Although a considerable amount of research on yield performance is being performed little is known about the molecular networks underpinning yield (Van Camp, 2005; Gonzalez et al.2009). Many genes have been described in *Arabidopsis thaliana* that, when mutated or ectopically expressed, result in the formation of larger structures, such as leaves or roots. However, notwithstanding extensive research, the effect of overexpression of a gene, and especially the effect of expression of combinations of genes is unpredictable. There is still need for further genes that can be used to increase yield, especially genes that have an additive or even synergetic effect with other growth promoting genes.

The small auxin-up RNA (SAUR) family comprises a large set of genes whose expressions are early auxin-responsive (Franco et al., 1990; Anai et al., 1998; Jain et al., 2006). However, the function of these genes is largely unknown. Knauss et al. (2002) indicate that SAUR2 of Zea *mays* encodes a short lived nuclear protein that might be involved in auxin-mediated cell elongation. Park et al. (2006) showed that SAUR32 of Arabidopsis is involved in apical hook development. Recently, Kant et al. (2009) demonstrated that SAUR 39 of *Oryza sativa* acts as a negative regulator of auxin synthesis and transport in rice. WO2008061240 discloses the use of SAUR22 of *Arabidopsis thaliana* and other members of the SAUR family to improve cold tolerance; however, no data are shown.

Surprisingly we found that expression of a SAUR fusion protein, but not overexpression of SAUR itself is promoting plant growth. Even more surprisingly only N-terminal fusions to SAUR, and not the C-terminal fusion show the plant growth promoting effect. Interestingly, the growth promoting effect of SAUR can be combined with the effect of other growth promoting genes, resulting in an additive or even synergetic effect on plant growth. This effect is rather exceptional, A as most combinations of growth promoting genes give a less than additive effect, or even a smaller effect for the combination of genes than for each individual growth promoting gene.

Disclosed herein is the use of a fusion protein comprising a SAUR polypeptide selected from the group consisting of SAUR 19, SAUR 21, SAUR 23 and SAUR 24 wherein said heterologous polypeptide is fused to the N-terminal end of said SAUR polypeptide and wherein said fusion protein is expressed in combination with a protein selected from the group consisting of ARL (genbank accession number: NP_850409.1), ANT (AAB17364.1), AGF1 (NP_195265.2), APC10 (Q9ZPW2.2), GRF5(NP_568325.1) and AVP1 (NP_001077542.1), and/or in combination with the expression of the JAW gene encoding a microRNA (AY922344.1), and/or in combination with a downregulated or inactivated gene encoding DA1-1 (NP_173361.1), preferably a DA1-1 knock out. An inactivated DA1-1 gene as used here refers also to DA1-1 genes encoding a defective, or less active DA1-1 protein. Preferably the increase or plant growth and/or yield, obtained by the coexpression of the genes, is at least additive, preferably said increase is synergetic. Most preferably, said SAUR polypeptide comprises a sequence selected from the group consisting of SEQ ID N°1-4 (sequences tested).

SAUR proteins are known to the person skilled in the art, and include but are not limited to the genes listed in table 1, and homologues or orthologues thereof. The terms "polypeptide" and "protein" are used interchangeably herein and refer to amino acids in a polymeric form of any length, linked together by peptide bonds. A "fusion protein" refers to a protein wherein two polypeptides which in nature do not occur together as part of the same protein are linked to each other by a peptide bond to form one protein.

Increase of plant growth and/or yield is measured by comparing the test plant, coexpressing a gene encoding a fusion protein as described in this application with a gene selected from the group consisting of ARL, ANT, AGF1, APC10, GRF5 and AVP1, and/or in combination with the expression of a recombinant microRNA encoded by JAW, and/or in combination with a downregulated or inactivated DA1-1 gene, with the parental, non-transformed plant, grown under the same conditions as control. Preferably, increase of growth is measured as an increase of biomass production. "Yield" refers to a situation where only a part of the plant, preferably an economical important part of the plant, such as the leaves, roots or seeds, is increased in biomass. The term "increase" as used here means least a 5%, 6%, 7%, 8%, 9% or 10%, preferably at least 15% or 20%, more preferably 25%, 30%, 35% or 40% more yield and/or growth in comparison to control plants as defined herein. Increase of plant growth, as used here, is preferably measured as increase of any one or more of total plant biomass, leaf biomass, root biomass and seed biomass. An example of said increase is an increase in total plant biomass. Preferably, said use is the expression of the fusion protein in said plant. Preferably, said plant is selected from the group consisting of *Arabidopsis thaliana, Brassicus* sp., *Glycine max, Medicago truncatula, Vitis vinifera, Populus sp., Solanum* sp., *Beta vulgaris, Gossypium hirsutum, Avena sativa, Hordeum vulgare, Triticum aestivum, Oryza sativa, Phyllostachys edulis, Miscanthus* sp., *Panicum virgatum, Zea mays, Saccharum officinarum, Sorghum bicolor* and *Ricinus communis.* Said plant can be a crop plant, preferably a monocot or a cereal, even more preferably it is a cereal selected from the group consisting of rice, maize, wheat, barley, millet, rye, sorghum and oats.

The application also discloses a transgenic plant, comprising a fusion protein according to the application, wherein said transgenic plant further comprises a recombinant gene encoding a protein selected from the group consisting of ARL, ANT, AGF1, APC10, GRF5 and AVP1, and/or comprises a recombinant gene encoding an inactive DA1-1 and/or a recombinant JAW gene encoding a microRNA. Preferably said transgenic plant is selected from the group consisting of *Arabidopsis thaliana, Brassicus* sp., *Glycine max, Medicago truncatula, Vitis vinifera, Populus sp., Solanum* sp., *Beta vulgaris, Gossypium hirsutum, Avena sativa, Hordeum vulgare, Triticum aestivum, Oryza sativa, Phyllostachys edulis, Miscanthus* sp., *Panicum virgatum, Zea mays, Saccharum officinarum, Sorghum bicolor* and *Ricinus communis.* Said plant can be a crop plant, preferably a monocot or a cereal, even more preferably it is a cereal selected from the group consisting of rice, maize, wheat, barley, millet, rye, sorghum and oats.

In this application, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the application, all those constructions brought about by recombinant methods in which either (a) the nucleic acid sequences encoding proteins useful in the methods of the application, or (b) genetic control sequence(s) which is operably linked with the nucleic acid sequence according to the application, for example a promoter, or (c) a) and b) are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present application, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815. The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

A transgenic plant for the purposes of the application is thus understood as meaning, as above, that the nucleic acids used in the method of the application are not at their natural locus in the genome of said plant, it being possible for the nucleic acids to be expressed homologously or heterologously. However, as mentioned, transgenic also means that, while the nucleic acids according to the application or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the application at an unnatural locus in the genome, i.e. homologous or, preferably, heterologous expression of the nucleic acids takes place.

The application also discloses a method for obtaining plants with increased growth characteristics, comprising (1) isolating a nucleic acid encoding a polypeptide of the SAUR family, wherein said SAUR is selected from the group consisting of SAUR19, SAUR21, SAUR23 and SAUR24, (2) fusing said nucleic acid at the 5' end to a nucleic acid encoding a heterologous peptide, wherein said fusion results in a N-terminal fusion to the SAUR polypeptide (3) transforming the fused nucleic acid into a plant. Preferably, said fused nucleic acid is operably linked to a suitable promoter. "Operably linked" refers to a juxtaposition wherein the components so described are in a relationship permitting them to function in their intended manner. A promoter sequence "operably linked" to a coding sequence is ligated in such a way that expression of the coding sequence is achieved under conditions compatible with the promoter sequence. Preferably, said plant is selected from the group consisting of *Arabidopsis thaliana, Brassicus* sp., *Glycine max, Medicago truncatula, Vitis vinifera, Populus sp., Solanum* sp., *Beta vulgaris, Gossypium hirsutum, Avena sativa, Hordeum vulgare, Triticum aestivum, Oryza sativa, Phyllostachys edulis, Miscanthus* sp., *Panicum virgatum, Zea mays, Saccharum officinarum, Sorghum bicolor* and *Ricinus communis.* Said plant can be a crop plant, preferably a monocot or a cereal, preferably it is a cereal selected from the group consisting of rice, maize, wheat, barley, millet, rye, sorghum and oats. Preferably, said SAUR polypeptide comprises a sequence selected from the group consisting of SEQ ID N°1-4 (sequences tested).

**Table I: non-limiting list of members of the SAUR family**

| *Species* | *length* | *accession number* | |
|---|---|---|---|
| SAUR [Raphanus sativus] | 95 aa | BAA25434.1 | GI:3043536 |
| SAUR family protein [Musa balbisiana] | 176 aa | BAG70988.1 | GI:199601729 |
| SAUR family protein [Musa balbisiana] | 176 aa | BAG70998.1 | GI:199601704 |
| SAUR33[Zea mays] | 131 aa | NP_001159042.1 | GI:259490681 |
| SAUR12[Zea mays] | 125 aa | NP_001151012.1 | GI:226533132 |
| SAUR55[Zea mays] | 149 aa | NP_001148334.1 | GI:226531724 |
| SAUR52[Zea mays] | 152 aa | NP_001151993.1 | GI:226530832 |
| SAUR20[Zea mays] | 166 aa | NP_001148938.1 | GI:226530568 |
| SAUR37[Zea mays] | 157 aa | NP_001147560.1 | GI:226529857 |
| SAUR40[Zea mays] | 89 aa | NP_001152402.1 | GI:226528649 |
| SAUR31[Zea mays] | 102 aa | NP_001148413.1 | GI:226510187 |
| SAUR23[Zea mays] | 161 aa | NP_001151649.1 | GI:226510085 |
| SAUR25[Zea mays] | 139 aa | NP_001148237.1 | GI:226509128 |
| SAUR20[Zea mays] | 169 aa | NP_001150569.1 | GI:226507274 |
| SAUR30[Zea mays] | 117 aa | NP_001151597.1 | GI:226506882 |
| SAUR11[Zea mays] | 202 aa | NP_001151006.1 | GI:226506308 |
| SAUR16[Zea mays] | 108 aa | NP_001147394.1 | GI:226505984 |
| SAUR11[Zea mays] | 199 aa | NP_001151756.1 | GI:226505124 |
| SAUR1[Zea mays] | 103 aa | NP_001152203.1 | GI:226504722 |
| SAUR52[Zea mays] | 147 aa | NP_001150183.1 | GI:226503087 |
| SAUR14[Zea mays] | 103 aa | NP_001147398.1 | GI:226501774 |
| SAUR56[Zea mays] | 127 aa | NP_001150182.1 | GI:226499078 |
| SAUR33[Zea mays] | 137 aa | NP_001146880.1 | GI:226494413 |
| SAUR9[Zea mays] | 107 aa | NP_001147174.1 | GI:226492799 |
| SAUR family protein [Populus trichocarpa] | 118 aa | EEF06097.1 | GI:222868966 |
| SAUR family protein [Populus trichocarpa] | 125 aa | EEF05238.1 | GI:222868107 |
| SAUR family protein [Populus trichocarpa] | 123 aa | EEF05232.1 | GI:222868101 |
| SAUR family protein [Populus trichocarpa] | 144 aa | EEF04069.1 | GI:222866938 |
| SAUR family protein [Populus trichocarpa] | 160 aa | EEF03893.1 | GI:222866762 |
| SAUR family protein [Populus trichocarpa] | 119 aa | EEF03856.1 | GI:222866725 |
| SAUR family protein [Populus trichocarpa] | 128 aa | EEF03855.1 | GI:222866724 |
| SAUR family protein [Populus trichocarpa] | 148 aa | EEF03854.1 | GI:222866723 |
| SAUR family protein [Populus trichocarpa] | 167 aa | EEF03289.1 | GI:222866158 |
| SAUR family protein [Populus trichocarpa] | 153 aa | EEF03223.1 | GI:222866092 |
| SAUR family protein [Populus trichocarpa] | 185 aa | EEF02601.1 | GI:222865470 |
| SAUR family protein [Populus trichocarpa] | 131 aa | EEF02550.1 | GI:222865419 |
| SAUR family protein [Populus trichocarpa] | 156 aa | EEF01541.1 | GI:222864410 |
| SAUR family protein [Populus trichocarpa] | 121 aa | EEF01490.1 | GI:222864359 |
| SAUR family protein [Populus trichocarpa] | 177 aa | EEF00366.1 | GI:222862859 |
| SAUR family protein [Populus trichocarpa] | 110 aa | EEE98534.1 | GI:222860992 |
| SAUR family protein [Populus trichocarpa] | 170 aa | EEE98363.1 | GI:222860821 |
| SAUR family protein [Populus trichocarpa] | 125 aa | EEE98233.1 | GI:222860686 |
| SAUR family protein [Populus trichocarpa] | 181 aa | EEE96973.1 | GI:222859426 |
| SAUR family protein [Populus trichocarpa] | 135 aa | EEE96685.1 | GI:222859138 |
| SAUR family protein [Populus trichocarpa] | 97 aa | EEE95603.1 | GI:222858056 |
| SAUR family protein [Populus trichocarpa] | 104 aa | EEE94704.1 | GI:222857157 |
| SAUR family protein [Populus trichocarpa] | 149 aa | EEE93913.1 | GI:222856366 |
| SAUR family protein [Populus trichocarpa] | 123 aa | EEE93715.1 | GI:222856168 |
| SAUR family protein [Populus trichocarpa] | 136 aa | EEE93401.1 | GI:222855854 |
| SAUR family protein [Populus trichocarpa] | 124 aa | EEE92944.1 | GI:222855397 |
| SAUR family protein [Populus trichocarpa] | 149 aa | EEE91612.1 | GI:222854065 |
| SAUR family protein [Populus trichocarpa] | 104 aa | EEE91306.1 | GI:222853759 |
| SAUR family protein [Populus trichocarpa] | 114 aa | EEE90949.1 | GI:222853402 |
| SAUR family protein [Populus trichocarpa] | 138 aa | EEE90544.1 | GI:222852997 |
| SAUR family protein [Populus trichocarpa] | 136 aa | EEE89259.1 | GI:222851712 |
| SAUR family protein [Populus trichocarpa] | 132 aa | EEE88450.1 | GI:222850903 |
| SAUR family protein [Populus trichocarpa] | 146 aa | EEE87704.1 | GI:222850157 |
| SAUR family protein [Populus trichocarpa] | 98 aa | EEE87702.1 | GI:222850155 |
| SAUR family protein [Populus trichocarpa] | 68 aa | EEE87701.1 | GI:222850154 |
| SAUR family protein [Populus trichocarpa] | 141 aa | EEE87700.1 | GI:222850153 |
| SAUR family protein [Populus trichocarpa] | 104 aa | EEE86957.1 | GI:222849410 |
| SAUR family protein [Populus trichocarpa] | 91 aa | EEE86955.1 | GI:222849408 |
| SAUR family protein [Populus trichocarpa] | 92 aa | EEE86954.1 | GI:222849407 |
| SAUR family protein [Populus trichocarpa] | 99 aa | EEE86953.1 | GI:222849406 |
| SAUR family protein [Populus trichocarpa] | 98 aa | EEE86951.1 | GI:222849404 |
| SAUR family protein [Populus trichocarpa] | 145 aa | EEE86950.1 | GI:222849403 |
| SAUR family protein [Populus trichocarpa] | 93 aa | EEE86949.1 | GI:222849402 |
| SAUR family protein [Populus trichocarpa] | 95 aa | EEE86948.1 | GI:222849401 |
| SAUR family protein [Populus trichocarpa] | 97 aa | EEE86947.1 | GI:222849400 |
| SAUR family protein [Populus trichocarpa] | 65 aa | EEE86946.1 | GI:222849399 |
| SAUR family protein [Populus trichocarpa] | 90 aa | EEE86945.1 | GI:222849398 |
| SAUR family protein [Populus trichocarpa] | 107 aa | EEE86944.1 | GI:222849397 |
| SAUR family protein [Populus trichocarpa] | 149 aa | EEE86883.1 | GI:222849336 |
| SAUR family protein [Populus trichocarpa] | 148 aa | EEE86882.1 | GI:222849335 |
| SAUR family protein [Populus trichocarpa] | 148 aa | EEE86881.1 | GI:222849334 |
| SAUR family protein [Populus trichocarpa] | 148 aa | EEE86880.1 | GI:222849333 |
| SAUR family protein [Populus trichocarpa] | 146 aa | EEE86879.1 | GI:222849332 |
| SAUR family protein [Populus trichocarpa] | 148 aa | EEE86729.1 | GI:222849182 |
| SAUR family protein [Populus trichocarpa] | 74 aa | EEE86671.1 | GI:222849124 |
| SAUR family protein [Populus trichocarpa] | 89 aa | EEE86670.1 | GI:222849123 |
| SAUR family protein [Populus trichocarpa] | 72 aa | EEE86669.1 | GI:222849122 |
| SAUR family protein [Populus trichocarpa] | 92 aa | EEE86668.1 | GI:222849121 |
| SAUR family protein [Populus trichocarpa] | 97 aa | EEE86667.1 | GI:222849120 |
| SAUR family protein [Populus trichocarpa] | 67 aa | EEE86666.1 | GI:222849119 |
| SAUR family protein [Populus trichocarpa] | 90 aa | EEE86665.1 | GI:222849118 |
| SAUR family protein [Populus trichocarpa] | 94 aa | EEE86664.1 | GI:222849117 |
| SAUR family protein [Populus trichocarpa] | 99 aa | EEE86663.1 | GI:222849116 |
| SAUR family protein [Populus trichocarpa] | 99 aa | EEE86662.1 | GI:222849115 |
| SAUR family protein [Populus trichocarpa] | 96 aa | EEE86661.1 | GI:222849114 |
| SAUR family protein [Populus trichocarpa] | 98 aa | EEE86660.1 | GI:222849113 |
| SAUR family protein [Populus trichocarpa] | 104 aa | EEE86658.1 | GI:222849111 |
| SAUR family protein [Populus trichocarpa] | 91 aa | EEE85987.1 | GI:222848440 |
| SAUR family protein [Populus trichocarpa] | 98 aa | EEE85986.1 | GI:222848439 |
| SAUR family protein [Populus trichocarpa] | 101 aa | EEE85985.1 | GI:222848438 |
| SAUR family protein [Populus trichocarpa] | 145 aa | EEE85982.1 | GI:222848435 |
| SAUR family protein [Populus trichocarpa] | 106 aa | EEE85019.1 | GI:222847472 |
| SAUR family protein [Populus trichocarpa] | 160 aa | EEE84134.1 | GI:222846587 |
| SAUR family protein [Populus trichocarpa] | 127 aa | EEE83807.1 | GI:222846260 |
| SAUR family protein [Populus trichocarpa] | 150 aa | EEE81433.1 | GI:222843886 |
| SAUR family protein [Populus trichocarpa] | 149 aa | EEE81260.1 | GI:222843713 |
| SAUR family protein [Populus trichocarpa] | 100 aa | EEE81154.1 | GI:222843607 |
| SAUR family protein [Populus trichocarpa] | 111 aa | EEE80702.1 | GI:222843155 |
| SAUR family protein [Populus trichocarpa] | 171 aa | EEE80545.1 | GI:222842998 |
| SAUR family protein [Populus trichocarpa] | 94 aa | EEE80141.1 | GI:222842594 |
| SAUR family protein [Populus trichocarpa] | 104 aa | EEE79990.1 | GI:222842443 |
| SAUR family protein [Populus trichocarpa] | 126 aa | EEE79223.1 | GI:222841676 |
| SAUR family protein [Populus trichocarpa] | 167 aa | EEE78749.1 | GI:222841202 |
| SAUR family protein [Populus trichocarpa] | 169 aa | EEE78501.1 | GI:222840954 |
| SAUR family protein [Populus trichocarpa] | 106 aa | EEE78304.1 | GI:222840757 |
| SAUR protein [Medicago truncatula] | 125 aa | ABE80127.1 | GI:92870927 |
| SAUR protein [Medicago truncatula] | 140 aa | ABD32679.1 | GI:87240821 |
| SAUR protein [Medicago truncatula] | 130 aa | ABD32678.1 | GI:87240820 |
| SAUR family protein [Populus trichocarpa] | 109 aa | XP_002337465.1 | GI:224154353 |
| SAUR family protein [Populus trichocarpa] | 144 aa | XP_002336416.1 | GI:224147130 |
| SAUR family protein [Populus trichocarpa] | 177 aa | XP_002325984.1 | GI:224146373 |
| SAUR family protein [Populus trichocarpa] | 167 aa | XP_002324724.1 | GI:224142769 |
| SAUR family protein [Populus trichocarpa] | 153 aa | XP_002324658.1 | GI:224142631 |
| SAUR family protein [Populus trichocarpa] | 144 aa | XP_002323936.1 | GI:224141147 |
| SAUR family protein [Populus trichocarpa] | 160 aa | XP_002323760.1 | GI:224140787 |
| SAUR family protein [Populus trichocarpa] | 119 aa | XP_002323723.1 | GI:224140713 |
| SAUR family protein [Populus trichocarpa] | 128 aa | XP_002323722.1 | GI:224140711 |
| SAUR family protein [Populus trichocarpa] | 148 aa | XP_002323721.1 | GI:224140709 |
| SAUR family protein [Populus trichocarpa] | 125 aa | XP_002323477.1 | GI:224140209 |
| SAUR family protein [Populus trichocarpa] | 123 aa | XP_002323471.1 | GI:224140197 |
| SAUR family protein [Populus trichocarpa] | 81 aa | XP_002327252.1 | GI:224135575 |
| SAUR family protein [Populus trichocarpa] | 118 aa | XP_002321970.1 | GI:224135047 |
| SAUR family protein [Populus trichocarpa] | 110 aa | XP_002320219.1 | GI:224128011 |
| SAUR family protein [Populus trichocarpa] | 170 aa | XP_002320048.1 | GI:224127334 |
| SAUR family protein [Populus trichocarpa] | 105 aa | XP_002329465.1 | GI:224126755 |
| SAUR family protein [Populus trichocarpa] | 97 aa | XP_002319680.1 | GI:224125808 |
| SAUR family protein [Populus trichocarpa] | 166 aa | XP_002329903.1 | GI:224125140 |
| SAUR family protein [Populus trichocarpa] | 181 aa | XP_002318753.1 | GI:224122102 |
| SAUR family protein [Populus trichocarpa] | 169 aa | XP_002330775.1 | GI:224121228 |
| SAUR family protein [Populus trichocarpa] | 135 aa | XP_002318465.1 | GI:224120972 |
| SAUR family protein [Populus trichocarpa] | 135 aa | XP_002331213.1 | GI:224119654 |
| SAUR family protein [Populus trichocarpa] | 125 aa | XP_002317621.1 | GI:224117608 |
| SAUR family protein [Populus trichocarpa] | 185 aa | XP_002316430.1 | GI:224113231 |
| SAUR family protein [Populus trichocarpa] | 131 aa | XP_002316379.1 | GI:224113065 |
| SAUR family protein [Populus trichocarpa] | 156 aa | XP_002315370.1 | GI:224109962 |
| SAUR family protein [Populus trichocarpa] | 121 aa | XP_002315319.1 | GI:224109812 |
| SAUR family protein [Populus trichocarpa] | 146 aa | XP_002313749.1 | GI:224105269 |
| SAUR family protein [Populus trichocarpa] | 98 aa | XP_002313747.1 | GI:224105265 |
| SAUR family protein [Populus trichocarpa] | 68 aa | XP_002313746.1 | GI:224105263 |
| SAUR family protein [Populus trichocarpa] | 141 aa | XP_002313745.1 | GI:224105261 |
| SAUR family protein [Populus trichocarpa] | 104 | XP_002313002.1 | GI:224103303 |
| SAUR family protein [Populus trichocarpa] | 91 aa | XP_002313000.1 | GI:224103297 |
| SAUR family protein [Populus trichocarpa] | 92 aa | XP_002312999.1 | GI:224103295 |
| SAUR family protein [Populus trichocarpa] | 99 aa | XP_002312998.1 | GI:224103293 |
| SAUR family protein [Populus trichocarpa] | 98 aa | XP_002312996.1 | GI:224103287 |
| SAUR family protein [Populus trichocarpa] | 145 aa | XP_002312995.1 | GI:224103283 |
| SAUR family protein [Populus trichocarpa] | 93 aa | XP_002312994.1 | GI:224103279 |
| SAUR family protein [Populus trichocarpa] | 95 aa | XP_002312993.1 | GI:224103277 |
| SAUR family protein [Populus trichocarpa] | 97 aa | XP_002312992.1 | GI:224103275 |
| SAUR family protein [Populus trichocarpa] | 65 aa | XP_002312991.1 | GI:224103273 |
| SAUR family protein [Populus trichocarpa] | 90 aa | XP_002312990.1 | GI:224103269 |
| SAUR family protein [Populus trichocarpa] | 107 aa | XP_002312989.1 | GI:224103265 |
| SAUR family protein [Populus trichocarpa] | 149 aa | XP_002312928.1 | GI:224103107 |
| SAUR family protein [Populus trichocarpa] | 148 aa | XP_002312927.1 | GI:224103103 |
| SAUR family protein [Populus trichocarpa] | 148 aa | XP_002312926.1 | GI:224103101 |
| SAUR family protein [Populus trichocarpa] | 148 aa | XP_002312925.1 | GI:224103099 |
| SAUR family protein [Populus trichocarpa] | 146 aa | XP_002312924.1 | GI:224103097 |
| SAUR family protein [Populus trichocarpa] | 136 aa | XP_002311892.1 | GI:224100477 |
| SAUR family protein [Populus trichocarpa] | 132 aa | XP_002311083.1 | GI:224097848 |
| SAUR family protein [Populus trichocarpa] | 89 aa | XP_002334603.1 | GI:224097550 |
| SAUR family protein [Populus trichocarpa] | 104 aa | XP_002310856.1 | GI:224097158 |
| SAUR family protein [Populus trichocarpa] | 114 aa | XP_002310499.1 | GI:224095881 |
| SAUR family protein [Populus trichocarpa] | 138 aa | XP_002310094.1 | GI:224094216 |
| SAUR family protein [Populus trichocarpa] | 124 aa | XP_002309421.1 | GI:224091969 |
| SAUR family protein [Populus trichocarpa] | 149 aa | XP_002308089.1 | GI:224087160 |
| SAUR family protein [Populus trichocarpa] | 104 aa | XP_002307708.1 | GI:224085816 |
| SAUR family protein [Populus trichocarpa] | 149 aa | XP_002306917.1 | GI:224082982 |
| SAUR family protein [Populus trichocarpa] | 123 aa | XP_002306719.1 | GI:224082502 |
| SAUR family protein [Populus trichocarpa] | 136 aa | XP_002306405.1 | GI:224081427 |
| SAUR family protein [Populus trichocarpa] | 148 aa | XP_002306218.1 | GI:224080736 |
| SAUR family protein [Populus trichocarpa] | 74 aa | XP_002306160.1 | GI:224080554 |
| SAUR family protein [Populus trichocarpa] | 89 aa | XP_002306159.1 | GI:224080550 |
| SAUR family protein [Populus trichocarpa] | 72 aa | XP_002306158.1 | GI:224080548 |
| SAUR family protein [Populus trichocarpa] | 92 aa | XP_002306157.1 | GI:224080546 |
| SAUR family protein [Populus trichocarpa] | 97 aa | XP_002306156.1 | GI:224080544 |
| SAUR family protein [Populus trichocarpa] | 67 aa | XP_002306155.1 | GI:224080540 |
| SAUR family protein [Populus trichocarpa] | 90 aa | XP_002306154.1 | GI:224080536 |
| SAUR family protein [Populus trichocarpa] | 94 aa | XP_002306153.1 | GI:224080532 |
| SAUR family protein [Populus trichocarpa] | 99 aa | XP_002306152.1 | GI:224080530 |
| SAUR family protein [Populus trichocarpa] | 99 aa | XP_002306151.1 | GI:224080528 |
| SAUR family protein [Populus trichocarpa] | 96 aa | XP_002306150.1 | GI:224080524 |
| SAUR family protein [Populus trichocarpa] | 98 aa | XP_002306149.1 | GI:224080522 |
| SAUR family protein [Populus trichocarpa] | 104 aa | XP_002306147.1 | GI:224080516 |
| SAUR family protein [Populus trichocarpa] | 91 aa | XP_002305476.1 | GI:224078022 |
| SAUR family protein [Populus trichocarpa] | 98 aa | XP_002305475.1 | GI:224078016 |
| SAUR family protein [Populus trichocarpa] | 101 aa | XP_002305474.1 | GI:224078010 |
| SAUR family protein [Populus trichocarpa] | 145 aa | XP_002305471.1 | GI:224077988 |
| SAUR family protein [Populus trichocarpa] | 126 aa | XP_002304244.1 | GI:224074081 |
| SAUR family protein [Populus trichocarpa] | 167 aa | XP_002303770.1 | GI:224072526 |
| SAUR family protein [Populus trichocarpa] | 169 aa | XP_002303522.1 | GI:224071573 |
| SAUR family protein [Populus trichocarpa] | 106 aa | XP_002303325.1 | GI:224071003 |
| SAUR family protein [Populus trichocarpa] | 128 aa | XP_002326368.1 | GI:224069539 |
| SAUR family protein [Populus trichocarpa] | 122 aa | XP_002326359.1 | GI:224069496 |
| SAUR family protein [Populus trichocarpa] | 126 aa | XP_002326308.1 | GI:224069236 |
| SAUR family protein [Populus trichocarpa] | 160 aa | XP_002326143.1 | GI:224068552 |
| SAUR family protein [Populus trichocarpa] | 150 aa | XP_002302160.1 | GI:224066607 |
| SAUR family protein [Populus trichocarpa] | 149 aa | XP_002301987.1 | GI:224065947 |
| SAUR family protein [Populus trichocarpa] | 100 aa | XP_002301881.1 | GI:224065607 |
| SAUR family protein [Populus trichocarpa] | 111 aa | XP_002301429.1 | GI:224064344 |
| SAUR family protein [Populus trichocarpa] | 171 aa | XP_002301272.1 | GI:224063729 |
| SAUR family protein [Populus trichocarpa] | 94 aa | XP_002300868.1 | GI:224062657 |
| SAUR family protein [Populus trichocarpa] | 104 aa | XP_002300717.1 | GI:224062025 |
| SAUR family protein [Populus trichocarpa] | 106 aa | XP_002300214.1 | GI:224060467 |
| SAUR family protein [Populus trichocarpa] | 160 aa | XP_002299329.1 | GI:224057798 |
| SAUR family protein [Populus trichocarpa] | 127 aa | XP_002299002.1 | GI:224056745 |
| auxin-induced SAUR 1 [Antirrhinum majus] | 48 aa | AAL55415.1 | GI:18071492 |
| SAUR family protein [Populus trichocarpa] | 89 aa | EEF10620.1 | GI:222873489 |
| SAUR family protein [Populus trichocarpa] | 135 aa | EEF10465.1 | GI:222873334 |
| SAUR family protein [Populus trichocarpa] | 169 aa | EEF09708.1 | GI:222872577 |
| SAUR family protein [Populus trichocarpa] | 166 aa | EEF08271.1 | GI:222871140 |
| SAUR family protein [Populus trichocarpa] | 105 aa | EEF07276.1 | GI:222870145 |
| SAUR family protein [Populus trichocarpa] | 81 aa | EEE74057.1 | GI:222835622 |
| SAUR family protein [Populus trichocarpa] | 128 aa | EEE72038.1 | GI:222833561 |
| SAUR family protein [Populus trichocarpa] | 122 aa | EEE72029.1 | GI:222833552 |
| SAUR family protein [Populus trichocarpa] | 126 aa | EEE71978.1 | GI:222833501 |
| SAUR family protein [Populus trichocarpa] | 160 aa | EEE71813.1 | GI:222833336 |
| SAUR family protein [Populus trichocarpa] | 109 aa | EEE77750.1 | GI:222839413 |
| SAUR family protein [Populus trichocarpa] | 144 aa | EEE73392.1 | GI:222834943 |
| SAUR12 [Zea mays] | 117 aa | ACG48743.1 | GI:195658551 |
| SAUR33 [Zea mays] | 135 aa | ACG48640.1 | GI:195658345 |
| SAUR40 [Zea mays] | 89 aa | ACG47423.1 | GI:195655911 |
| SAUR1 [Zea mays] | 103 aa | ACG46362.1 | GI:195653789 |
| SAUR52 [Zea mays] | 152 aa | ACG45294.1 | GI:195651653 |
| SAUR36 [Zea mays] | 145 aa | ACG45154.1 | GI:195651373 |
| SAUR37 [Zea mays] | 160 aa | ACG44891.1 | GI:195650847 |
| SAUR11 [Zea mays] | 181 aa | ACG44091.1 | GI:195649247 |
| SAUR23 [Zea mays] | 161 aa | ACG43656.1 | GI:195648376 |
| SAUR30 [Zea mays] | 117 aa | ACG43481.1 | GI:195648026 |
| SAUR55 [Zea mays] | 158 aa | ACG41822.1 | GI:195644708 |
| SAUR20 [Zea mays] | 169 aa | ACG39599.1 | GI:195640262 |
| SAUR44 [Zea mays] | 149 aa | ACG38928.1 | GI:195638920 |
| SAUR56 [Zea mays] | 127 aa | ACG38150.1 | GI:195637364 |
| SAUR55 [Zea mays] | 149 aa | ACG35907.1 | GI:195628154 |
| SAUR31 [Zea mays] | 102 aa | ACG31378.1 | GI:195619096 |
| SAUR25 [Zea mays] | 139 aa | ACG30255.1 | GI:195616850 |
| SAUR14 [Zea mays] | 103 aa | ACG27349.1 | GI:195611038 |
| SAUR16 [Zea mays] | 108 aa | ACG27315.1 | GI:195610970 |
| SAUR25 [Zea mays] | 138 aa | ACG26269.1 | GI:195608878 |
| SAUR9 [Zea mays] | 107 aa | ACG25834.1 | GI:195608008 |
| SAUR_C [Arabidopsis thaliana] | 189 aa | NP_194860.1 | GI:15235917 |
| SAUR_D [Arabidopsis thaliana] | 178 aa | NP_180016.1 | GI:15224133 |
| SAUR_B [Arabidopsis thaliana] | 190 aa | NP_001031914.1 | GI:79328260 |
| SAUR_E [Arabidopsis thaliana] | 150 aa | NP_195201.1 | GI:15236186 |
| [Arabidopsis thaliana] | 160 aa | NP_189898.1 | GI:15229222 |
| [Oryza sativa Japonica Group] | 166 aa | BAG98524.1 | GI:215766296 |
| [Oryza sativa Japonica Group] | 153 aa | BAG98478.1 | GI:215766250 |
| [Oryza sativa Japonica Group] | 128 aa | BAG98236.1 | GI:215766008 |
| [Oryza sativa Japonica Group] | 143 aa | BAG98212.1 | GI:215765984 |
| [Oryza sativa Japonica Group] | 120 aa | BAG87305.1 | GI:215765608 |
| [Oryza sativa Japonica Group] | 141 aa | BAG97924.1 | GI:215741429 |
| [Oryza sativa Japonica Group] | 133 aa | BAG97805.1 | GI:215741310 |
| [Oryza sativa Japonica Group] | 96 aa | BAG88609.1 | GI:215693227 |
| [Arabidopsis thaliana] | 112 aa | NP_179392.1 | GI:15227953 |
| [Arabidopsis thaliana] | 108 aa | NP_179248.1 | GI:15227246 |
| [Arabidopsis thaliana] | 104 aa | NP_179718.1 | GI:15226486 |
| [Arabidopsis thaliana] | 98 aa | NP_179717.1 | GI:15226485 |
| [Arabidopsis thaliana] | 86 aa | NP_179716.1 | GI:15226484 |
| [Arabidopsis thaliana] | 142 aa | NP_200171.2 | GI:42568515 |
| [Arabidopsis thaliana] | 146 aa | NP_173471.2 | GI:30686846 |
| [Arabidopsis thaliana] | 135 aa | NP_181163.2 | GI:30686707 |
| [Arabidopsis thaliana] | 131 aa | NP_849679.1 | GI:30685273 |
| [Arabidopsis thaliana] | 93 aa | NP_187035.2 | GI:30678959 |
| [Arabidopsis thaliana] | 131 aa | NP_683527.1 | GI:22330829 |
| [Arabidopsis thaliana] | 122 aa | NP_567196.1 | GI:18411465 |
| [Arabidopsis thaliana] | 154 aa | NP_565113.1 | GI:18410889 |
| [Arabidopsis thaliana] | 119 aa | NP_565042.1 | GI:18410081 |
| [Arabidopsis thaliana] | 124 aa | NP_565665.1 | GI:18401625 |
| [Arabidopsis thaliana] | 139 aa | NP_566440.1 | GI:18399805 |
| SAUR68 [Arabidopsis thaliana] | 143 aa | NP_564332.1 | GI:18397123 |
| [Arabidopsis thaliana] | 102 aa | NP_564331.1 | GI:18397116 |
| [Arabidopsis thaliana] | 148 aa | NP_564330.1 | GI:18397103 |
| [Arabidopsis thaliana] | 141 aa | NP_564329.1 | GI:18397101 |
| [Arabidopsis thaliana] | 114 aa | NP_195951.1 | GI:15242718 |
| [Arabidopsis thaliana] | 127 aa | NP_197582.1 | GI:15242071 |
| [Arabidopsis thaliana] | 183 aa | NP_199889.1 | GI:15241259 |
| [Arabidopsis thaliana] | 142 aa | NP_198130.1 | GI:15241052 |
| [Arabidopsis thaliana] | 99 aa | NP_201427.1 | GI:15239314 |
| [Arabidopsis thaliana] | 111 aa | NP_199056.1 | GI:15238955 |
| [Arabidopsis thaliana] | 148 aa | NP_196660.1 | GI:15238919 |
| [Arabidopsis thaliana] | 90 aa | NP_197309.1 | GI:15238736 |
| [Arabidopsis thaliana] | 90 aa | NP_197307.1 | GI:15238721 |
| [Arabidopsis thaliana] | 90 aa | NP_197306.1 | GI:15238719 |
| [Arabidopsis thaliana] | 88 aa | NP_197304.1 | GI:15238716 |
| [Arabidopsis thaliana] | 91 aa | NP_197303.1 | GI:15238715 |
| [Arabidopsis thaliana] | 90 aa | NP_197302.1 | GI:15238714 |
| [Arabidopsis thaliana] | 92 aa | NP_193115.1 | GI:15236351 |
| [Arabidopsis thaliana] | 105 aa | NP_195207.1 | GI:15236200 |
| [Arabidopsis thaliana] | 94 aa | NP_195206.1 | GI:15236199 |
| [Arabidopsis thaliana] | 108 aa | NP_195205.1 | GI:15236198 |
| [Arabidopsis thaliana] | 106 aa | NP_195204.1 | GI:15236189 |
| [Arabidopsis thaliana] | 104 aa | NP_195203.1 | GI:15236188 |
| [Arabidopsis thaliana] | 107 aa | NP_195202.1 | GI:15236187 |
| [Arabidopsis thaliana] | 160 aa | NP_193993.1 | GI:15235723 |
| [Arabidopsis thaliana] | 105 aa | NP_195597.1 | GI:15234829 |
| SAUR15 [Arabidopsis thaliana] | 89 aa | NP_195596.1 | GI:15234827 |
| [Arabidopsis thaliana] | 99 aa | NP_195595.1 | GI:15234825 |
| [Arabidopsis thaliana] | 157 aa | NP_192978.1 | GI:15234550 |
| [Arabidopsis thaliana] | 104 aa | NP_195334.1 | GI:15234294 |
| [Arabidopsis thaliana] | 103 aa | NP_192691.1 | GI:15233907 |
| [Arabidopsis thaliana] | 113 aa | NP_187598.1 | GI:15232781 |
| [Arabidopsis thaliana] | 170 aa | NP_191628.1 | GI:15232400 |
| [Arabidopsis thaliana] | 109 aa | NP_190893.1 | GI:15231777 |
| [Arabidopsis thaliana] | 118 aa | NP_188657.1 | GI:15231081 |
| [Arabidopsis thaliana] | 132 aa | NP_187889.1 | GI:15230601 |
| [Arabidopsis thaliana] | 106 aa | NP_190688.1 | GI:15230423 |
| [Arabidopsis thaliana] | 95 aa | NP_187034.1 | GI:15228641 |
| [Arabidopsis thaliana] | 92 aa | NP_187033.1 | GI:15228640 |
| [Arabidopsis thaliana] | 96 aa | NP_187032.1 | GI:15228639 |
| [Arabidopsis thaliana] | 136 aa | NP_191749.1 | GI:15228626 |
| [Arabidopsis thaliana] | 124 aa | NP_181240.1 | GI:15228072 |
| [Arabidopsis thaliana] | 121 aa | NP_182192.1 | GI:15226425 |
| [Arabidopsis thaliana] | 162 aa | NP_182046.1 | GI:15225432 |
| [Arabidopsis thaliana] | 153 aa | NP_173413.1 | GI:15223691 |
| [Arabidopsis thaliana] | 117 aa | NP_173411.1 | GI:15223685 |
| [Arabidopsis thaliana] | 110 aa | NP_176011.1 | GI:15223462 |
| [Arabidopsis thaliana] | 123 aa | NP_177746.1 | GI:15222984 |
| [Arabidopsis thaliana] | 108 aa | NP_177688.1 | GI:15222294 |
| [Arabidopsis thaliana] | 134 aa | NP_178034.1 | GI:15219296 |
| [Arabidopsis thaliana] | 147 aa | NP_173100.1 | GI:15219275 |
| [Arabidopsis thaliana] | 135 aa | NP_174243.1 | GI:15218951 |
| [Arabidopsis thaliana] | 141 aa | NP_174236.1 | GI:15218924 |
| [Arabidopsis thaliana] | 104 aa | NP_175002.1 | GI:15218238 |
| Os09g0437100 [Oryza sativa Japonica] | 165 aa | BAH94585.1 | GI:255678926 |
| Os06g0702000 [Oryza sativa Japonica] | 61 aa | BAH93710.1 | GI:255677370 |
| Os06g0701900 [Oryza sativa Japonica] | 134 aa | BAF20401.2 | GI:255677369 |
| Os06g0671150 [Oryza sativa Japonica] | 140 aa | BAH93678.1 | GI:255677311 |
| Os04g0617050 [Oryza sativa Japonica] | 120 aa | BAH92818.1 | GI:255675777 |
| Os04g0517900 [Oryza sativa Japonica] | 129 aa | BAH92744.1 | GI:255675624 |
| Os01g0924966 [Oryza sativa Japonica] | 173 aa | BAH91446.1 | GI:255674015 |
| Os01g0768333 [Oryza sativa Japonica] | 122 aa | BAH91311.1 | GI:255673718 |
| Os02g0306200 [Oryza sativa Japonica] | 143 aa | BAH91643.1 | GI:255670825 |
| Os02g0143400 [Oryza sativa Japonica] | 130 aa | BAF07783.2 | GI:255670598 |
| Os02g0143300 [Oryza sativa Japonica] | 120 aa | BAF07782.2 | GI:255670597 |
| Os12g0626200 [Oryza sativa Japonica] | 130 aa | BAF30342.1 | GI:113649830 |
| Os10g0510500 [Oryza sativa Japonica] | 125 aa | BAF26951.1 | GI:113639646 |
| Os09g0546100 [Oryza sativa Japonica] | 141 aa | BAF25753.1 | GI:113632072 |
| Os09g0437400 [Oryza sativa Japonica] | 190 aa | BAF25178.1 | GI:113631497 |
| Os08g0452500 [Oryza sativa Japonica] | 133 aa | BAF23868.1 | GI:113623923 |
| Os08g0118500 [Oryza sativa Japonica] | 109 aa | BAF22782.1 | GI:113622837 |
| Os07g0475700 [Oryza sativa Japonica] | 120 aa | BAF21536.1 | GI:113611158 |
| Os06g0714300 [Oryza sativa Japonica] | 141 aa | BAF20487.1 | GI:113596613 |
| Os04g0662400 [Oryza sativa Japonica] | 153 aa | BAF16070.1 | GI:113565727 |
| Os04g0662200 [Oryza sativa Japonica] | 143 aa | BAF16069.1 | GI:113565726 |
| Os02g0769100 [Oryza sativa Japonica] | 128 aa | BAF10155.1 | GI:113537772 |
| Os02g0643800 [Oryza sativa Japonica] | 190 aa | BAF09475.1 | GI:113537092 |
| Os02g0512000 [Oryza sativa Japonica] | 166 aa | BAF08854.1 | GI:113536471 |
| Os02g0445600 [Oryza sativa Japonica] | 94 aa | BAF08663.1 | GI:113536280 |
| Os02g0445100 [Oryza sativa Japonica] | 96 aa | BAF08662.1 | GI:113536279 |
| [Oryza sativa Japonica Group] | 90 aa | BAH01591.1 | GI:215769362 |
| [Oryza sativa Japonica Group] | 129 aa | BAH01004.1 | GI:215768775 |
| [Oryza sativa Japonica Group] | 171 aa | BAH00993.1 | GI:215768764 |
| [Oryza sativa Japonica Group] | 141 aa | BAH00901.1 | GI:215768672 |
| [Oryza sativa Japonica Group] | 176 aa | BAG99230.1 | GI:215767002 |
| [Oryza sativa Japonica Group] | 173 aa | BAG99136.1 | GI:215766908 |
| [Oryza sativa Japonica Group] | 150 aa | BAG86730.1 | GI:215765033 |
| [Oryza sativa Japonica Group] | 133 aa | BAG95554.1 | GI:215734832 |
| [Oryza sativa Japonica Group] | 141 aa | BAG88424.1 | GI:215693004 |
| [Arabidopsis thaliana] | 99 aa | CAB80547.1 | GI:7270867 |
| Os12g0626200 [Oryza sativa] | 130 aa | NP_001067323.1 | GI:115489672 |
| Os10g0510500 [Oryza sativa] | 125 aa | NP_001065037.1 | GI:115482888 |
| Os09g0547000 [Oryza sativa] | 141 aa | NP_001063846.1 | GI:115480505 |
| Os09g0546300 [Oryza sativa] | 157 aa | NP_001063841.1 | GI:115480495 |
| Os09g0546000 [Oryza sativa] | 144 aa | NP_001063838.1 | GI:115480489 |
| Os09g0508100 [Oryza sativa] | 138 aa | NP_001063619.1 | GI:115480051 |
| Os09g0437400 [Oryza sativa] | 190 aa | NP_001063264.1 | GI:115479341 |
| Os08g0452500 [Oryza sativa] | 133 aa | NP_001061954.1 | GI:115476716 |
| Os08g0118500 [Oryza sativa] | 109 aa | NP_001060868.1 | GI:115474543 |
| Os07g0475700 [Oryza sativa] | 120 aa | NP_001059622.1 | GI:115472047 |
| Os06g0714300 [Oryza sativa] | 141 aa | NP_001058573.1 | GI:115469948 |
| Os06g0701900 [Oryza sativa] | 312 aa | NP_001058487.1 | GI:115469776 |
| Os04g0662400 [Oryza sativa] | 153 aa | NP_001054156.1 | GI:115461112 |
| Os04g0662200 [Oryza sativa] | 143 aa | NP_001054155.1 | GI:115461110 |
| Os04g0608300 [Oryza sativa] | 176 aa | NP_001053812.1 | GI:115460424 |
| Os02g0769100 [Oryza sativa] | 128 aa | NP_001048241.1 | GI:115448923 |
| Os02g0643800 [Oryza sativa] | 190 aa | NP_001047561.1 | GI:115447563 |
| Os02g0512000 [Oryza sativa] | 166 aa | NP_001046940.1 | GI:115446321 |
| Os02g0445600 [Oryza sativa] | 94 aa | NP_001046749.1 | GI:115445939 |
| Os02g0445100 [Oryza sativa] | 96 aa | NP_001046748.1 | GI:115445937 |
| Os02g0143400 [Oryza sativa] | 124 aa | NP_001045869.1 | GI:115444179 |
| Os02g0143300 [Oryza sativa] | 121 aa | NP_001045868.1 | GI:115444177 |
| [Vigna radiata var. radiata] | 92 aa | BAA03310.1 | GI:287570 |
| [Vigna radiata var. radiata] | 196 aa | BAA03309.1 | GI:287568 |
| [Vigna radiata var. radiata] | 194 aa | BAA03308.1 | GI:287566 |
| SAUR-AC-like protein [Arabidopsis thaliana] | 92 aa | CAB78421.1 | GI:7268083 |

### BRIEF DESCRIPTION OF THE FIGURES

**Figure 1****:** Hypocotyl length of 10 days old (do) light grown seedlings. N-terminal fusions of GFP or Strepll with SAUR19 and SAUR24 confer increased hypocotyl length (1) Col control; (2) N-terminal GFP-SAUR 19 fusion; (3) N-terminal Strepll-SAUR19 fusion; (4) N-terminal Strepll-SAUR24 fusion.
**Figure 2****:** Hypocotyl length of 10 do light grown seedlings. N-terminal GFP fusions of SAUR19, 21, 23, or 24 confer increased hypocotyl length. Untagged or C-terminally tagged SAUR19 constructs resemble Col controls. (1) Col control; (2) N-terminal GFP-SAUR19 fusion; (3) N-terminal GFP-SAUR21 fusion; (4) N-terminal GFP-SAUR23 fusion; (5) N-terminal GFP-SAUR24 fusion; (6) overexpression of non-fused SAUR 19; (7) C-terminal SAUR19-GFP fusion
**Figure 3****:** Biomass measurements. The vegetative part of plants (Col Controls and GFP-SAUR19 plants) grown *in vitro* was harvested 20 days after stratification and fresh weigth was measured (n=12). Two independent lines are analyzed (SAUR19-1 and SAUR19-2)
**Figure 4****:** Leaf area measurements. Plants were grown in vitro for 20 days and leaf series were done to measure individual leaf area. Green: GFP- SAUR19 OE lines (two independent lines: SAUR19-1 and SAUR19-2), orange: wild type plants.
**Figure 5****:** Combination of GFP-SAUR19 overexpression with other IYG lines. A/ example of a combination leading to an additive effect on leaf area. B/ example of a combination leading to a positive effect (more than expected) on growth. For a given cross, the leaf area expected in case of an additive effect (*) was calculated by adding the areas of the two heterozygous parents and subtracting the area from the control Col-0.

### EXAMPLES

### Materials and methods to the examples

### SAUR DNA constructs

SAUR coding sequences were PCR amplified from Arabidopsis Col-0 genomic DNA and Gateway cloned into pDONR207. The resulting donor clones were sequenced and then used to transfer the SAUR coding regions into destination vectors pMDC32(Curtiss and Grossniklaus, 2003) (35S::SAUR), pMDC43(Curtiss and Grossniklaus, 2003) (35S::GFP-SAUR), pMDC84(Curtiss and Grossniklaus, 2003) (35S::SAUR-GFP), or pJ2B-StepII-GW (35S::StrepII-SAUR) as per the Gateway LR Clonase protocol (Invitrogen). The resulting binary vectors were introduced into Agrobacterium strain GV3101, which was used to transform Arabidopsis Col-0 plants using the floral dip method.

### Plant material and growth condition

Two independent homozygous lines (SAUR19-1 and SAUR19-2) overexpressing the SAUR19 (At5g18010) gene fused to the GFP at its N-terminal end were selected after transformation of agrobacterium with the construct containing the GFP-SAUR19 fusion under the control of the CaMV35S promoter. These plants were grown for phenotypic analysis in vitro in half-strength Murashige and Skoog medium (Murashige and Skoog, 1962), supplemented with 1% sucrose at 21 °C under a 16-h day/8-h night regime.

### Growth analysis

For the biomass measurement, the vegetative part of a 20 days old plant is harvested and fresh weight is measured.For the rosette leaf area measurements, 8-12 seedlings were grown under in vitro conditions for 20 days. Individual leaves (cotyledons and rosette leaves) were dissected and their area was measured with the ImageJ software (http://rsb.info.nih.gov/ij/).

### Hypocotyl assays

Homozygous transgenic lines were identified and transgene expression was confirmed by Northern hybridization. Seeds from homozygous transgenic lines were sterilized with 30% bleach, stratified at 4°C for four days, and then transferred to ATS(Lincoln et al., 1990) agar plates. Seedlings were grown for 10 days in a plant growth chamber at 20°C under long day (16:8) light conditions. On day 10, seedlings were photographed and hypocotyl lengths measured on a computer using imaging software.

### Combination of growth enhancing lines

To obtain the double transgenic plants, homozygous lines expressing the respective growth enhancing genes were crossed with each other and the F1 progeny analyzed. As controls, crosses were made with a Col-0 wild-type. Leaf series were performed from plants grown under in vitro conditions for 20 days and leaf area was measured with the ImageJ software (http://rsb.info.nih.gov/ij/).

### Example 1: Phenotypic analysis of overexpressors of SAUR fusion proteins

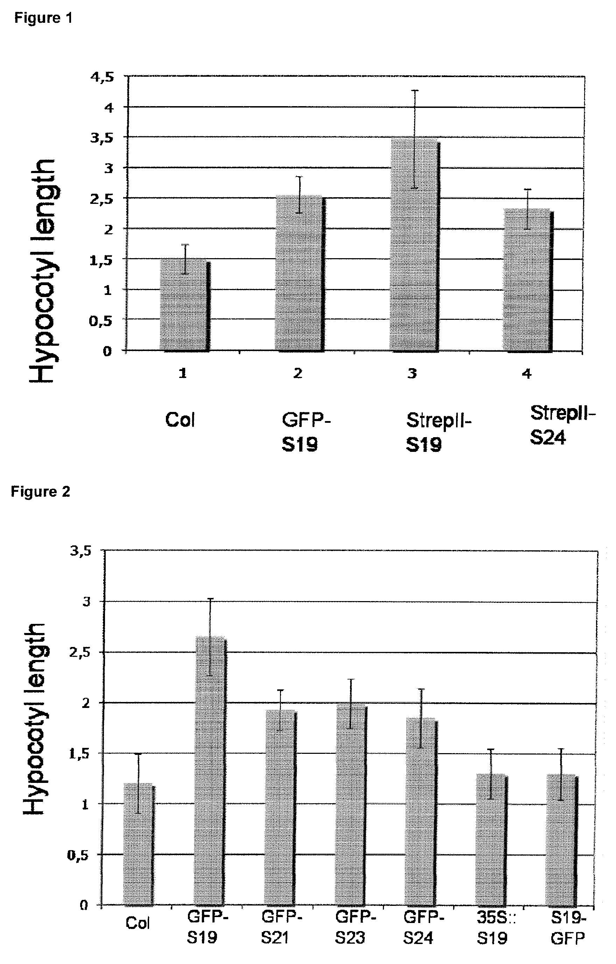

Plants expressing N-terminally tagged SAUR fusion proteins exhibited a 1.5 - 2X increase in hypocotyl length. This increase in organ size appears to be predominantly due to increased cell expansion, as measurements of cell length were highly correlated with hypocotyl length. In contrast, no effects on hypocotyl length were observed with plants overexpressing untagged or C-terminally-tagged SAUR proteins. A minimum of three independent transgenic lines were analyzed for each construct (Figure 1 and 2).

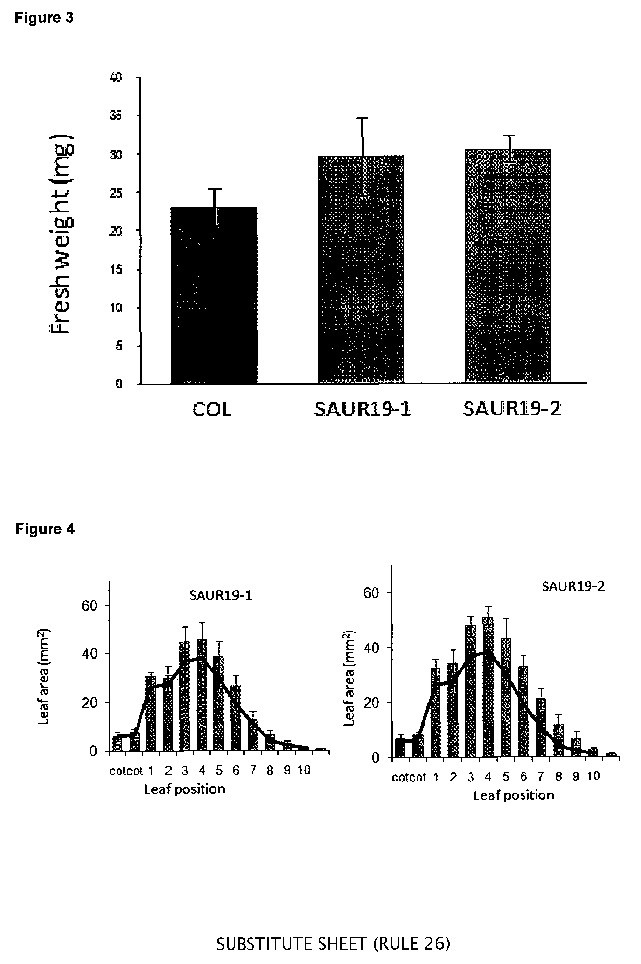

In order to evaluate the effect of the over-expression of SAUR 19 on growth, measurements of biomass and leaf area were performed on the GFP-SAUR19 lines. The vegetative part of plants grown under *in vitro* conditions was harvested 20 days after stratification and fresh weight was measured. As shown in figure 3, the biomass of the GFP-SAUR19 is increased by 30% when compared to the wild type plants. Subsequently leaf series were made from plants harvested at the same stage in order to measure the area of each individual leaf from the rosette (figure 4). This analysis revealed that the two SAUR19-GFP lines lead to the production of larger leaves, however, the extent of the increased size differs between the two lines. The GFP- SAUR19 (line 1) leads to an increase of the area of leaves 4 to 6 while in GFP- SAUR19 (line 2), almost all the leaves are larger (2 to 9).

### Example 2: Crosses with other IYG lines

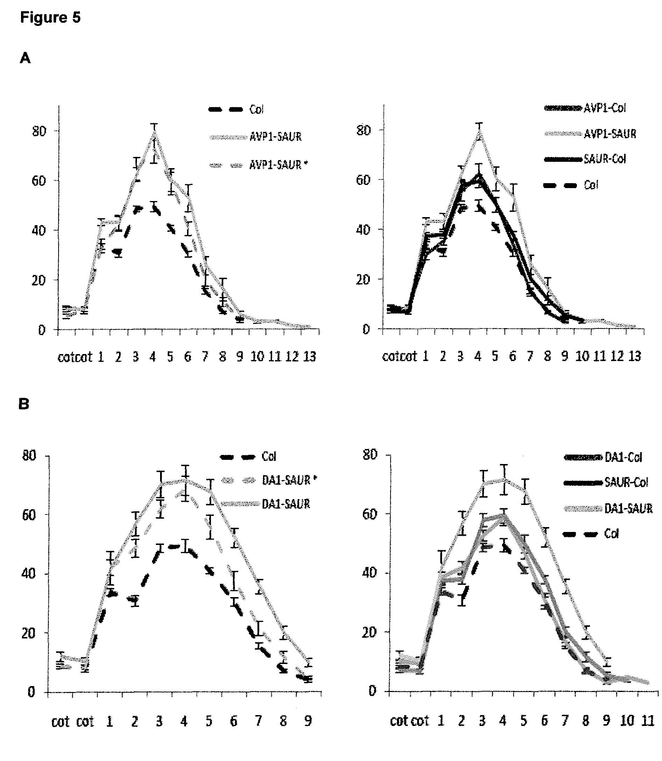

In order to investigate the potential genetic connections between SAUR19 and other genes leading to an increase in leaf area when over-expressed or down-regulated, crosses between GFP-SAUR19-2 and 10 Intrinsic Yield Genes (IYG) lines (AGF1 (WO02079403), ANT (Mizukami et al., 2000), APC10, ARL (Hu Y, et al. 2006), AVP1 (Li J, et al. 2005), BRI1 (Wang Z-Y, et al. 2001), DA1 (Li Y, et al. 2008) EXP10 (Cho H-T, Cosgrove DJ. 2000), GA20OX (Coles JP, et al. 1999), GRF5 (Horiguchi, et al. 2005) and JAW (Schommer C, et al. 2008)) were performed. IYG are genes that when overexpressed or mutated enhance plant growth. Growth behaviour of the heterozygous progeny was analyzed under standard conditions by measuring leaf area. This analysis allowed identifying additive/synergistic/antagonistic effects of gene combinations on growth. For example, when GFP-SAUR19 and AVP1 simultanously are overexpressed in the same plant, the final leaf size corresponds to the sum of the effect of the heterozygous parents suggesting that these genes work independently to control organ size (figure5A). Interestingly, the combination GFP-SAUR19 and DA1-1 leads to the production of leaves that are larger than what is expected for an additive effect, suggesting that these two genes work synergistically to increase leaf size (figure5B). In most of the cases the combination leads to an additive effect (AVP1, APC10, AGF1, ANT, ARL, JAW, GRF5). In one case, GFP-SAUR19 combined with BRI, we observed a negative effect on leaf growth. For two cases, the combination of GFP-SAUR19 with EXP10 and GA20-ox, the effect on growth is unclear.

### REFERENCES

- Anai, T., Kono, N., Kosemura, S., Yamamura, S. and Hasegawa, K. (1998). Isolation and characterization of an auxsin-inducilbe SAUR gene from radish seedlings. DNA seq. 9, 329-333.
- Cho HT, Cosgrove DJ (2000) Altered expression of expansin modulates leaf growth and pedicel abscission in Arabidopsis thaliana. Proc Natl Acad Sci U S A 97: 9783-9788.
- Coles JP, Phillips AL, Croker SJ, Garcia-Lepe R, Lewis MJ, Hedden P (1999) Modification of gibberellin production and plant development in Arabidopsis by sense and antisense expression of gibberellin 20-oxidase genes. Plant J 17: 547-556.
- Curtis, M.D. and Grossniklaus, U. (2003). A gateway cloning vector set for high-throughput functional analysis of genes in planta. Plant Physiol 133, 462-469.
- Franco, A.R., Gee, M.A. and Guilfoyle, T.J. (1990). Induction and superinduction of auxin-responsive mRNAs with auxin and protein synthesis inhibitors. J. Biol. Chem.. 265, 15845-15849.
- Gonzalez, N. Gerrit TS Beemster, G.T.S. and Inzé D. (2009).David and Goliath: what can the tiny weed Arabidopsis teach us to improve biomass production in crops? Current Opinion in Plant Biology. 12: 157-164.
- Horiguchi G, Kim GT, Tsukaya H (2005) The transcription factor AtGRF5 and the transcription coactivator AN3 regulate cell proliferation in leaf primordia of Arabidopsis thaliana. Plant J 43: 68-78.
- Hu Y, Poh HM, Chua NH (2006) The Arabidopsis ARGOS-LIKE gene regulates cell expansion during organ growth. Plant J 47: 1-9.
- Jain, M., Tyagi, A.K. and Khurana, J.P. (2006). Genome-wide analysis, evolutionary expansion, and expression of early auxin-responsive SAUR gene family in rice (Oryzae sativa). Genomics, 88, 360-371.
- Kant, S., Bi, Y.M., Zhu, T and Rothstein, S.J. (2009). SAUR39, a small auxin-up RNA gene, acts as a negative regulator of auxin synthesis and transport in rice. Plant Physiol. 151, 691-701.
- Li J, Yang H, Peer WA, Richter G, Blakeslee J, Bandyopadhyay A, Titapiwantakun B, Undurraga S, Khodakovskaya M, Richards EL, Krizek B, Murphy AS, Gilroy S, Gaxiola R (2005) Arabidopsis H+-PPase AVP1 regulates auxin-mediated organ development. Science 310: 121-125.
- Li Y, Zheng L, Corke F, Smith C, Bevan MW (2008) Control of final seed and organ size by the DA1 gene family in Arabidopsis thaliana. Genes Dev 22: 1331-1336.
- Knauss, S., Rohrmeier, T. and Lehle, L. (2003). The auxin-induced maize gene ZmSAUR2 encodes short-lived nuclear protein expressed in elongating tissues.
- Lincoln, C., Britton, J.H. and Estelle, M. (1990). Growth and development of the axr1 mutants of Arabidopsis. Plant Cell 2, 1071-1080.
- Marchler-Bauer, A., Anderson, J.B., Chitsaz, F., Derbyshire, M.K., DeWeese-Scott, C., Fong, J.H., Geer, L.Y., Geer, R.C., Gonzales, N.R., Gwadz, M., He, S., Hurwitz, D.I., Jackson, J.D., Ke, Z., Lanczycki, C.J., Liebert, C.A., Liu, C., Lu, F., Lu, S., Marchler, G.H., Mullokandov, M., Song, J.S., Tasneem, A., Thanki, N., Yamashita, R.A., Zhang, D., Zhang, N. and Bryant, S.H. (2009). CDD: specific functional annotation with the Conserved Domain Database. Nucleic Acids Res. 37(Database issue):D205-10.
- Mizukami Y, Fischer RL (2000) Plant organ size control: AINTEGUMENTA regulates growth and cell numbers during organogenesis. Proc Natl Acad Sci U S A 97: 942-947.
- Murashige T, Skoog F (1962) A revised medium for rapid growth and bio assays with tobacco tissue cultures. Physiol Plant 15: 473 497
- Palatnik JF, Allen E, Wu X, Schommer C, Schwab R, Carrington JC, Weigel D (2003) Control of leaf morphogenesis by microRNAs. Nature 425: 257-263.
- Park J-E, Kim Y-S, Yoon H-K, Park C-M (2006) Functional characterization of a small auxin-up RNA gene in apical hook development in Arabidopsis. Plant Science 172: 150-157.
- Van Camp, W. (2005). Yield enhancement genes: seeds for growth. Curr Opin Biotech 16: 147-153
- Wang ZY, Seto H, Fujioka S, Yoshida S, Chory J (2001) BRI1 is a critical component of a plasma-membrane receptor for plant steroids. Nature 410: 380-383.

## Claims

1. The use of a fusion protein comprising a SAUR polypeptide selected from the group consisting of SAUR 19, SAUR 21, SAUR 23 and SAUR 24, wherein said heterologous polypeptide is fused to the N-terminal end of SAUR and wherein said fusion protein is expressed in combination of the expression of a recombinant gene encoding a protein selected from the group consisting of ARL, ANT, AGF1, APC10, GRF5 and AVP1, and/or in combination with the expression of a recombinant microRNA encoded by JAW, and/or in combination with a downregulated or inactivated DA1-1 gene.

2. The use of a fusion protein according to claim 1, wherein said fusion protein comprises a sequence selected from the group consisting of SEQ ID N° 1-4.

3. The use of a fusion protein according to any of the claims 1 or 2 to increase plant growth and/or plant yield.

4. A transgenic plant, comprising a fusion protein comprising a SAUR polypeptide selected from the group consisting of SAUR 19, SAUR 21, SAUR 23 and SAUR 24, wherein said heterologous polypeptide is fused to the N-terminal end of SAUR, wherein said fusion protein increases plant growth and/or plant yield, said transgenic plant further comprising a recombinant gene encoding an inactive DA1-1 and/or a recombinant gene encoding protein selected from the group consisting of ARL, ANT, AGF1, APC10, GRF5 and AVP1, and/or a recombinant JAW gene encoding a microRNA.

5. The transgenic plant according to claim 4, wherein said plant is selected from the group consisting of *Arabidopsis thaliana, Brassicus* sp., *Glycine max, Medicago truncatula, Vitis vinifera, Populus sp., Solanum* sp., *Beta vulgaris, Gossypium hirsutum, Avena sativa, Hordeum vulgare, Triticum aestivum, Oryza sativa, Phyllostachys edulis, Miscanthus* sp., *Panicum virgatum, Zea mays, Saccharum officinarum, Sorghum bicolor* and *Ricinus communis.*

6. A method for obtaining plants with increased growth characteristics, comprising (1) isolating a nucleic acid encoding a SAUR polypeptide selected from the group consisting of SAUR 19, SAUR 21, SAUR 23 and SAUR 24, (2) fusing said nucleic acid at the 5' end to a nucleic acid encoding a heterologous peptide, wherein said fusion results in a N-terminal fusion to the SAUR polypeptide, wherein said fusion protein increases plant growth and/or plant yield, (3) transforming the fused nucleic acid into a plant and (4) expressing said fusion polypeptide in a plant comprising a recombinant gene encoding an inactive DA1-1 and/or a recombinant gene encoding protein selected from the group consisting of ARL, ANT, AGF1, APC10, GRF5 and AVP1, and/or a recombinant JAW gene encoding a microRNA.

7. The method according to claim 6, wherein said plant is selected from the group consisting of *Arabidopsis thaliana, Brassicus* sp., *Glycine max, Medicago truncatula, Vitis vinifera, Populus sp., Solanum* sp., *Beta vulgaris, Gossypium hirsutum, Avena sativa, Hordeum vulgare, Triticum aestivum, Oryza sativa, Phyllostachys edulis, Miscanthus* sp., *Panicum virgatum, Zea mays, Saccharum officinarum, Sorghum bicolor* and *Ricinus communis.*

## Patentansprüche

1. Verwendung eines Fusionsproteins, das ein SAUR-Polypeptid umfasst, das aus der Gruppe bestehend aus SAUR 19, SAUR 21, SAUR 23 und SAUR 24 ausgewählt ist, wobei das heterologe Polypeptid mit dem N-terminalen Ende von SAUR fusioniert ist und wobei das Fusionsprotein in Kombination mit der Expression eines rekombinanten Gens, das ein Protein kodiert, das aus der Gruppe bestehend aus ARL, ANT, AGF1, APC10, GRF5 und AVP1 ausgewählt ist, und/oder in Kombination mit der Expression einer rekombinanten MikroRNA, die von JAW kodiert wird, und/oder in Kombination mit einem herunterregulierten oder inaktivierten DA1-1-Gen exprimiert wird.

2. Verwendung eines Fusionsproteins nach Anspruch 1, wobei das Fusionsprotein eine Sequenz umfasst, die aus der Gruppe bestehend aus SEQ ID Nr. 1-4 ausgewählt ist.

3. Verwendung eines Fusionsproteins nach einem der Ansprüche 1 oder 2, um Pflanzenwachstum und/oder Pflanzenertrag zu erhöhen.

4. Transgene Pflanze, die ein Fusionsprotein umfasst, das ein SAUR-Polypeptid umfasst, das aus der Gruppe bestehend aus SAUR 19, SAUR 21, SAUR 23 und SAUR 24 ausgewählt ist, wobei das heterologe Polypeptid mit dem N-terminalen Ende von SAUR fusioniert ist, wobei das Fusionsprotein Pflanzenwachstum und/oder Pflanzenertrag erhöht, wobei die transgene Pflanze weiterhin ein rekombinantes Gen, das ein inaktives DA1-1 kodiert, und/oder ein rekombinantes Gen, das Protein kodiert, das aus der Gruppe bestehend aus ARL, ANT, AGF1, APC10, GRF5 und AVP1 ausgewählt ist, und/oder ein rekombinantes JAW-Gen, das eine MikroRNA kodiert, umfasst.

5. Transgene Pflanze nach Anspruch 4, wobei die Pflanze aus der Gruppe bestehend aus *Arabidopsis thaliana, Brassicus* sp., *Glycine max, Medicago truncatula, Vitis vinifera, Populus sp., Solanum* sp., *Beta vulgaris, Gossypium hirsutum, Avena sativa, Hordeum vulgare, Triticum aestivum, Oryza sativa, Phyllostachys edulis, Miscanthus* sp., *Panicum virgatum, Zea mays, Saccharum officinarum, Sorghum bicolor* und *Ricinus communis* ausgewählt ist.

6. Verfahren zum Erhalten von Pflanzen mit erhöhten Wachstumscharakteristika, das (1) das Isolieren einer Nukleinsäure, die ein SAUR-Polypeptid kodiert, das aus der Gruppe bestehend aus SAUR 19, SAUR 21, SAUR 23 und SAUR 24 ausgewählt ist, (2) das Fusionieren der Nukleinsäure an dem 5'-Ende mit einer Nukleinsäure, die ein heterologes Peptid kodiert, wobei die Fusion zu einer N-terminalen Fusion mit dem SAUR-Polypeptid führt, wobei das Fusionsprotein Pflanzenwachstum und/oder Pflanzenertrag erhöht, (3) das Transformieren der fusionierten Nukleinsäure zu einer Pflanze und (4) das Exprimieren des Fusionspolypeptids in einer Pflanze umfasst, die ein rekombinantes Gen, das ein inaktives DA1-1 kodiert, und/oder ein rekombinantes Gen, das Protein kodiert, das aus der Gruppe bestehend aus ARL, ANT, AGF1, APC10, GRF5 und AVP1 ausgewählt ist, und/oder ein rekombinantes JAW-Gen, das eine MikroRNA kodiert, umfasst.

7. Verfahren nach Anspruch 6, wobei die Pflanze aus der Gruppe bestehend aus *Arabidopsis thaliana, Brassicus* sp., *Glycine max, Medicago truncatula, Vitis vinifera, Populus sp., Solanum* sp., *Beta vulgaris, Gossypium hirsutum, Avena sativa, Hordeum vulgare, Triticum aestivum, Oryza sativa, Phyllostachys edulis, Miscanthus* sp., *Panicum virgatum, Zea mays, Saccharum officinarum, Sorghum bicolor* und *Ricinus communis* ausgewählt ist.

## Revendications

1. Utilisation d'une protéine de fusion comprenant un polypeptide SAUR sélectionné parmi le groupe constitué de SAUR 19, SAUR 21, SAUR 23 et SAUR 24, dans laquelle ledit polypeptide hétérologue est fusionné à l'extrémité N-terminale de SAUR et dans laquelle ladite protéine de fusion est exprimée en combinaison de l'expression d'un gène recombiné codant pour une protéine sélectionnée parmi le groupe constitué de ARL, ANT, AGF1, APC10, GRF5 et AVP1, et/ou en combinaison avec l'expression d'un microARN recombiné codé par JAW, et/ou en combinaison avec un gène DA1-1 régulé à la baisse ou inactivé.

2. Utilisation d'une protéine de fusion selon la revendication 1, dans laquelle ladite protéine de fusion comprend une séquence sélectionnée parmi le groupe constitué de SEQ ID N° 1 à 4.

3. Utilisation d'une protéine de fusion selon l'une quelconque des revendications 1 ou 2 pour augmenter la croissance végétale et/ou le rendement végétal.

4. Plante transgénique, comprenant une protéine de fusion comprenant un polypeptide SAUR sélectionné parmi le groupe constitué de SAUR 19, SAUR 21, SAUR 23 et SAUR 24, dans laquelle ledit polypeptide hétérologue est fusionné à l'extrémité N-terminale de SAUR, dans laquelle ladite protéine de fusion augmente la croissance végétale et/ou le rendement végétal, ladite plante transgénique comprenant en outre un gène recombiné codant pour un DA1-1 inactif et/ou un gène recombiné codant pour une protéine sélectionnée parmi le groupe constitué de ARL, ANT, AGF1, APC10, GRF5 et AVP1, et/ou un gène JAW recombiné codant pour un microARN.

5. Plante transgénique selon la revendication 4, dans laquelle ladite plante est sélectionnée parmi le groupe constitué de *Arabidopsis thaliana, Brassicus* sp., *Glycine max, Medicago truncatula, Vitis vinifera, Populus sp., Solanum* sp., *Beta vulgaris, Gossypium hirsutum, Avena sativa, Hordeum vulgare, Triticum aestivum, Oryza sativa, Phyllostachys edulis, Miscanthus* sp., *Panicum virgatum, Zea mays, Saccharum officinarum, Sorghum bicolor* et *Ricinus communis.*

6. Procédé d'obtention de plantes avec des caractéristiques de croissance accrue, comprenant (1) isoler un acide nucléique codant pour un polypeptide SAUR sélectionné parmi le groupe constitué de SAUR 19, SAUR 21, SAUR 23 et SAUR 24, (2) fusionner ledit acide nucléique à l'extrémité 5' à un acide nucléique codant pour un peptide hétérologue, dans lequel ladite fusion conduit à une fusion N-terminale au polypeptide SAUR, dans lequel ladite protéine de fusion augmente la croissance végétale et/ou le rendement végétal, (3) transformer l'acide nucléique fusionné en une plante et (4) exprimer ledit polypeptide de fusion dans une plante comprenant un gène recombiné codant pour un DA1-1 inactif et/ou un gène recombiné codant pour une protéine sélectionnée parmi le groupe constitué de ARL, ANT, AGF1, APC10, GRF5 et AVP1, et/ou un gène JAW recombiné codant pour un microARN.

7. Procédé selon la revendication 6, dans lequel ladite plante est sélectionnée parmi le groupe constitué de *Arabidopsis thaliana, Brassicus* sp., *Glycine max, Medicago truncatula, Vitis vinifera, Populus sp., Solanum* sp., *Beta vulgaris, Gossypium hirsutum, Avena sativa, Hordeum vulgare, Triticum aestivum, Oryza sativa, Phyllostachys edulis, Miscanthus* sp., *Panicum virgatum, Zea mays, Saccharum officinarum, Sorghum bicolor* et *Ricinus communis.*
